(19) Europäisches Patentamt
European Patent Office
Office européen des brevets

(11) Publication number: **0 022 330**
**B1**

(12) **EUROPEAN PATENT SPECIFICATION**

(45) Date of publication of patent specification: **21.12.83**

(21) Application number: **80302139.3**

(22) Date of filing: **26.06.80**

(51) Int. Cl.[3]: **A 61 K 45/06,**
A 61 K 31/55 //(A61K31/55, 31/54), (A61K31/55, 31/505), (A61K31/55, 31/38)

(54) **Synergistic antihypertensive compositions.**

(30) Priority: **10.07.79 US 56174**

(43) Date of publication of application:
**14.01.81 Bulletin 81/2**

(45) Publication of the grant of the patent:
**21.12.83 Bulletin 83/51**

(84) Designated Contracting States:
**AT BE CH DE FR GB IT LI LU NL SE**

(56) References cited:
**DE - A - 2 629 887**
**DE - A - 2 751 258**
**DE - A - 2 849 766**
**US - A - 3 515 786**

**ROTE LISTE 1977/78 Ed. Cantor Aulendorf/Württ DE, Page 16 088B "Sali-Presinol and page 16 089B" Sembrina-Saltucin**

**CHEMICAL ABSTRACTS, vol. 88, nr. 23, June 5, 1978, page 95, columns 1 and 2, abstract 164443q Columbus, Ohio, US LOWENSTEIN J. "Combination drug therapy of hypertension-vasodilators"**

(73) Proprietor: **SMITHKLINE BECKMAN CORPORATION**
**1 Franklin Plaza**
**Philadelphia Pennsylvania 19101 (US)**

(72) Inventor: **Weinstock, Joseph**
**1234 Pothouse Road**
**Phoenixville Pennsylvania 19406 (US)**
Inventor: **Wiebelhaus, Virgil Daniel**
**471 Lynnbrook Road**
**Springfield Pennsylvania 19064 (US)**

(74) Representative: **Waters, David Martin, Dr. et al,**
**Smith Kline & French Laboratories Ltd. Patent Department Mundells**
**Welwyn Garden City Hertfordshire AL7 1EY (GB)**

(56) References cited:

**Remington's Pharmaceutical Sciences, 15th. edition (1975) pages 780, 862, 868, 869**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 022 330 B1

Synergistic antihypertensive compositions

This invention comprises a synergistically active medicinal composition as hereinafter defined containing one or more compounds having renal vasodilating activity which does not elevate glomerular filtration, that is a renal dopamine-like agent having no substantial activity at the dopaminergic sites of reaction in the central nervous system, with one or more diuretic agents known to the art. The composition demonstrates potent diuretic and antihypertensive effects.

Documents of use in understanding the background of this invention are US Patent 3,515,786, German Patent specification 2,629,887, Chemical Abstracts *88,* 164443q (1978) and Rote Liste 1977/78 Nos 16088B and 16089B.

Renal dopamine-like agents which can be used are described in Belgian patent specification 860,774, German patent specification 2,849,766 or US patent specification 4,160,765. Diuretic agents which can be used are described in prior art mentioned hereafter in the specification.

The renal vasodilator ingredient is a compound of the Formula:

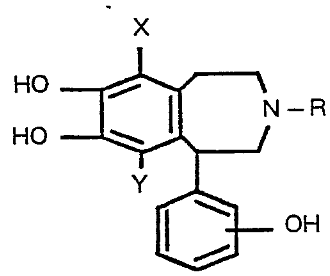

in which R is hydrogen or allyl, X is methyl carbomethoxy or halo and Y is hydrogen, methyl or halo. The phenolic hydroxy group in Formula I is preferably in the 4′-position. In one aspect Y is hydrogen and X is chloro.

Also included are derivatives of these compounds of Formula I which may give rise to the parent compounds *in vivo,* for example the pharmaceutically acceptable acid addition salts, e.g. hydrochloride, hydrobromide, sulfate, phosphate, sulfamate, methyl sulfonate, maleate or fumarate salts; O-esters especially the tri-O-loweralkanoyl esters having from 2—8 carbon atoms in each alkanoyl group; O-methyl ethers; and sulfate esters or glucuronate esters. Reference hereinafter to Formula I is intended to include reference to such esters and salts.

The amount of compound of Formula I present in the compositions of the invention will in general be calculated onn the free base form, the acid addition salts usually being most conveniently used, especially the hydrochloride, hydrobromide or methyl sulfonate salts. Prodrug and salt forms can be prepared by methods well known to the art.

The compounds of Formula I are described in the prior art as having an antihypertensive effect in subjects having, or susceptible to having, elevated blood pressure by means of a specific drug action at the peripheral dopaminergic receptors, especially in the kidney, thereby increasing renal blood flow and decreasing renal vascular resistance. The quantity of the renal dopaminergic agent in the compositions of this invention will be varied with the potency of its biological activity as well as with its pharmacodynamic characteristics. Generally, the quantity of renal dopaminergic agent in a dosage unit of this invention will be selected from the range of from about 15 to 500 mg. In another aspect the quantity of renal dopaminergic agent is 15 to 100 mg. Using, for example, 6 - chloro - 7,8 - dihydroxy - 1 - (*p* - hydroxyphenyl) - 2,3,4,5 - tetrahydro - 1H - 3 - benzazepines as its methyl sulfonate salt, from about 50—200 mg. of free base equivalent will usually be used with the diuretic agent.

The resulting composition will then be administered from 2—5 times a day. Other renal dopaminergic agents can be used, basing their unit dosages on this daily regimen.

In the test procedure for determining renal dopamine-like activity by infusion in anesthetized normotensive dogs, 6 - chloro - 7,8 - dihydroxy - 1 - (*p* - hydroxyphenyl) - 2,3,4,5 - tetrahydro - 1H - 3 - benzazepine has an $ED_{15}$ of 0.3 μg/kg. The details of the test procedure are presented in the literature references mentioned above. The tri-O-acetyl derivative of this compound has an $ED_{15}$ of 25 μg/kg, and the tri-O-isobutyryl derivative has an $ED_{15}$ of 16 μg/kg. Other compounds of Formula I having renal dopaminergic activity can be used by comparing their activity in the anesthetized dog protocol with that of the above specifically named compound in preparing hypotensively effective and non-toxic dosage regimens. For example, referring to compounds of the Formula I in which R is H:

| | | | |
|---|---|---|---|
| A. | X = Cl, Y = H, m-OH | $ED_{15}$ | 0.35 μg/kg. |
| B. | X = Br, Y = H, p-OH | $ED_{15}$ | 5 μg/kg. |
| C. | X = $CH_3$, Y = H, p-OH | $ED_{15}$ | 2.8 μg/kg. |

| | | | |
|---|---|---|---|
| D. | X = Cl, Y = Cl, p-OH | $ED_{15}$ 620 | μg/kg. |
| E. | X = $CH_3$, Y = Cl, p-OH | $ED_{15}$ 30 | μg/kg. |
| F. | X = I, Y = H, p-OH | $ED_{15}$ 4.2 | μg/kg. |
| G. | X = $CO_2CH_3$, Y = H, p-OH | $ED_{15}$ 2.3 | μg/kg. |

When R is allyl, X is Cl, Y is hydrogen and OH is in the 4′-position, the compound demonstrated activity at 0.3 μg/kg/min infusion rate.

The compounds of Formula I can be prepared as described in the literature or by methods outlined in the Examples.

The renal dopaminergic $ED_{15}$ values given above for the infused anesthetized dog are useful to assess quantitative and qualitative biological properties of the 1 - hydroxyphenyl - 7,8 - dihydroxy - 2,3,4,5 - tetrahydro - 1H - 3 - benzazepine compounds of Formula I. These compounds are preferably given by oral administration. They are rapidly absorbed from the gastrointestinal tract, and have a relatively short half life *in vivo*. Therefore, the renal dopaminergic agent must be matched with conventional dosage regimens of the diuretic agents considering the pharmacokinetic properties of each active component. This may be accomplished by combining the renal dopaminergic agent with a diuretic in standard dosage unit forms for administration from 2—6 times daily. This would be most suitable when the diuretic agent is of a short acting nature and conventionally multiple daily doses are used to give the desired maintenanance level.

Alternatively the renal dopaminergic agent may be incorporated into a timed release dosage unit form in which several doses are treated for delayed or sustained release of the medicament. Such dosage units may comprise sustained release granules, sugar centered spheres or multilayered tablets in each of which the availability of the active ingredient is controlled by coating with a lipid or polymeric material. In such compositions the diuretic component would usually be present in immediately available form.

The diuretic component of the antihypertensive compositions of this invention will be any diuretic agent known to the art and it will in general be present in the compositions in quantities within its normal dosage unit range and daily regimen range as detailed in the medical literature. Saluretic agents will normally be used. Normally the diuretic will be selected to give a daily quantity selected from about 1 mg to 1 g depending on its diuretic potency and mechanism of action. An effective quantity of the diuretic compound in a dosage unit of the present invention will usually be chosen from 0.5—500 mg., and preferably from 25—250 mg.

Known diuretic agents, their preferred dosage unit quantities in compositions of this invention, and the normal daily dose ranges are:

A. Diluting segment diuretics, for example:

| | | |
|---|---|---|
| chlorothiazide | 250, 500 mg | 500—1000 mg |
| hydrochlorothiazide | 25, 50 mg | 50—200 mg |
| cyclopenthiazide | 0.5 mg | 0.5—2 mg |
| bendrofluazide | 2.5, 5 mg | 2.5—10 mg |
| benzthiazide | 50 mg | 25—50 mg |
| butizide | 5 mg | 10 mg |
| cyclothiazide | 2 mg | 2—4 mg |
| hydroflumethiazide | 50 mg | 50—100 mg |
| methylclothiazide | 2.5, 5 mg | 2.5—10 mg |
| polythiazide | 1, 2, 4 mg | 1—4 mg |
| trichlormethiazide | 2, 4 mg | 4—8 mg |
| chlorthalidone | 25, 50 mg | 25—100 mg |
| quinethazone | 50 mg | 50—100 mg |
| clopamide | 20 mg | 20—80 mg |
| mefruside | 25 mg | 50 mg |
| clorexolone | 10—25 mg | 20—50 mg |

B. Distal nephron diuretics, for example:

| | | |
|---|---|---|
| spironolactone | 25, 50, 100 mg | 100—200 mg |
| triamterene | 50 mg | 100—250 mg |
| amiloride | 5 mg | 10—40 mg |
| 1,4-dimorpholino-7-phenylpyrido-[3,4-d] pyridazine | 10 mg | 5—100 mg |

C. Loop of Henle diuretics, for example:

| | | |
|---|---|---|
| furosemide | 40, 500 mg | 40—1000 mg |
| ethacrynic acid | 50, 100 mg | 100—400 mg |
| butmetanide | 1, 5 mg | 1—15 mg |
| [(6,7-dichloro-2,3-dihydro-2-methyl-1-oxo-2-phenyl-1H-indenyl-5-yl)oxy)] acetic acid (MK—196) | 10 mg | 10—30 mg |
| diapamide | 75 mg | — |
| 2-aminomethyl-4-(1,1-dimethylethyl)-6-iodophenol hydrochloride | 50 mg | |

D. Uricosuric Diuretics, for example:

| | | |
|---|---|---|
| ticrynafen | 100, 250, 500 mg | 100—1000 mg |
| 4(4-fluorobenzoyl)-2,3-dichlorophenoxy acetic acid | 100, 200 mg | — |
| 2-ethyl-3-(4-chloro-3-sulfamoyl-benzoyl) benzofuran | 10—500 mg | — |

The diuretic agents listed above are identified either by their generic names accepted by international or national health agencies or by their chemical names. The chemical structures, doses, and further biological characteristics including side effects are described in the literature [see for example K. H. Beyer, Clinical Therapeutics *1* 425—443 (1978) or T. O. Morgan, Drugs *15* 151—158 (1978)].

The active renal dopamine-like and diuretic components of the compositions of the invention will not usually be used in any particular ratio but each should be used at a clinically effective dose. The resulting compositions exhibit the full spectrum of the biological activity of the ingredients with unexpected synergistic effects.

The compositions of this invention can be prepared by standard pharmaceutical techniques by incorporation into a pharmaceutical carrier, and preferably in the form of a dosage unit, for example as a tablet, capsule, powder for suspension or solution or troche. For example an effective quantity of a renal dopaminergic compound of Formula I selected from the dose ranges presented above together with an effective quantity of diuretic agent selected from the dose ranges presented above are screened, sized and mixed together with a filler, if need be, for example talc, lactose, terra alba, magnesium stearate, agar, pectin, acacia, gelatin or stearic acid. The active ingredients and filler are mixed and filled into a hard gelatin capsule. Alternatively the ingredients are tabletted using lubricants and granulating agents. Also liquid carriers can be used, for example peanut oil, sesame oil, olive oil or water. Such mixtures can be encapsulated into soft gelatin capsules or, in case of a sterile, isotonic solution for parenteral use, in a unit or multidose vial.

If the renal dopaminergic agent is desired to be in a time release formulation, coating agents can be used, for example glyceryl distearate, wax, polyvinylpyrrolidone, zein, ethylcellulose, castor wax, polymethacrylates, cellulose acetate butyrate or a cross-linked polymeric film, for example one formed from prepolymers of an unsaturated dicarboxylic acid and an ethylene compound, or acid copolymers formed from acrylic or methacrylic acid monomers.

The compositions will in general be administered orally to patients who have abnormally high blood pressure or who are susceptible to have high blood pressure, the compositions usually being

administered from 1—6 times daily and preferably from 2—3 times daily. In certain cardiovascular emergencies the compositions containing the active ingredients in pharmaceutically acceptable salt form can be dissolved in saline when soluble and administered parenterally. It will be appreciated that the antihypertensive compositions of this invention combine clinically acceptable doses of a renal dopaminergic agent and a diuretic agent with consideration for the half life and peak therapeutic effect of each. It may be desirable in certain patients for the primary care physician to administer the two agents individually to achieve the novel therapeutic effect described herein. Also under certain circumstances, a third active agent selected from either class of compounds can be added, for example a combination of 6 - chloro - 1 - (*p* - hydroxyphenyl) - 7,8 - dihydroxy - 2,3,4,5 - tetrahydro - 1H - 3 - benzazepine methyl sulfonic acid salt, hydrochlorothiazide and triamterene can be used.

The compositions of the invention exhibit a synergistic effect on hypertension which is greater than the individual pharmacodynamic activity of either constituent of the compositions. The diuretic components of the compositions are saluretics which, although they may act at different portions of the nephron as described above, they have a diuretic threshold of activity. We postulate that the renal dopaminergic compound lowers the filtration fraction in the kidney by elevating renal plasma flow with little effect on the glomerular filtration rate, the latter being the key factor in the saluretic activity of diuretics. In addition to the unexpected fact that the renal dopaminergic agents of formula I do not interfere with the biological activity of the saluretic diuretics even though both groups which work by mechanisms within the kidney, an unexpected synergism between the two groups of compounds has been demonstrated.

In the glucose-infused unanesthetized dog, 6 - chloro - 7,8 - dihydroxy - 1 - (*p* - hydroxyphenyl) - 2,3,4,5 - tetrahydro - 1H - 3 - benzazepine methyl sulfonate was given at the peak time of natriureses established by a maximally effective dose of hydrochlorothiazide. The combination produced a significant increase in renal plasma flow (RPF), glomerular filtration rate (GFR) and $Na^+$ excretion over that observed with the thiazide alone. These results indicate that the capacity for the benzazepine to increase renal plasma flow in the dog is not affected by a natriuretic response produced by the distally acting diuretic, hydrochlorothiazide.

All dogs were given an oral water load of 3% of body weight of tap water thirty minutes prior to initiation of the experiment. Using a constant infusion pump the dogs were infused intravenously at 3 ml/min. throughout the course of the experiment, with a 5% dextrose solution containing 3.0 mg/kg/hr of hydrochlorothiazide (HCTZ). Glomerular filtration rate was determined by the clearance of creatinine and effective renal plasma flow by the clearance of *p*-aminohippuric acid (PAH). These clearances were determined simultaneously by including 0.4% creatinine and 0.08% *p*-aminohippuric acid in the infusion solution. The solution also contained 0.02% ascorbic acid. Suitable plasma levels of creatinine and *p*-aminohippuric acid were obtained by a twenty minute infusion of this solution to obtain equilibrium prior to starting of the initial urine collection. Also at the beginning of this 20 min period a stat i.v. injection of 2.5 mg/kg of hydrochlorothiazide in saline was given in addition to the 3.0 mg/kg/hr contained in the glucose infusion. 1.5 ml/kg of a 1% creatinine solution in saline was also injected intravenously fifteen minutes prior to the start of the collection of urine. Replicate urine collections were obtained at ten minute intervals throughout the experiment. The bladder was emptied by an indwelling catheter and complete urine collections were measured by rinsing the bladder with 10 ml of water at the end of each collection interval, followed by an air wash-out. Venous blood samples were drawn from an indwelling catheter at the mid-point of each clearance period.

Plasma and urine osmolalities were determined by freezing point depression on freshly obtained samples. Urine volume and pH were recorded at the time of collection. Plasma and urine samples were stored frozen after collection, until analyzed. Analysis for sodium and potassium were made using the flame photometer, and for chloride, creatinine, and p-aminohippuric acid, by colorimeter, using standard methodologies. These analyses were made simultaneously on each sample. Peak intensities were identified electronically, recorded by "on-line" teletype, stored and subsequently analyzed mathematically by time-shared computer against appropriate standards. Data were computed from linear least square regression lines and quality control standards were analyzed simultaneously to evaluate system reproducibility and reliability.

In the experiments where hydrochlorothiazide alone was given, twelve clearances were obtained. Data for the first 6 clearances were averaged and compared with the average of all parameters for clearances 7 to 12. In the combination study the benzazepine was given by stomach tube at 5.0 mg/kg (base) after $C_6$. The compound was solubilized in water with a small amount of sugar syrup to prevent or minimize emesis. Clearances $C_7$—$C_{12}$ (treatment) as in the hydrochlorothiazide alone tests, were taken with no wait between $C_6$ and $C_7$. Five dogs were used with both hydrochlorothiazide and with the combination.

TABLE I

The Effect of Hydrochlorothiazide and the Combination of Hydrochlorothiazide and 6-Chloro-7,8-dihydroxy-1-(*p*-hydroxyphenyl)-2,3,4,5-tetrahydro-1H-3-benzazepine methyl sulfonate in the Glucose Infused Dog

*Hydrochlorothiazide*

All values represent the difference between the averages of $C_1$—$C_6$ and $C_7$—$C_{12}$. Hydrochlorothiazide 2.5 mg/kg i.v. first then 3.0 mg/kg/hr infusion started at –20 min. and continued during $C_1$—$C_{12}$.

| Day | Ranal Plasma Flow (RPF) Δ % Change | Glomerular Filtration Rate (GFR) Δ % Change | Δ % $Na^+$ Excreted | Filtration Fraction Δ |
|---|---|---|---|---|
| 6 | –1 | –6 | –1.1 | –.01 |
| 7 | +6 | +6 | –0.8 | 0 |
| 9 | –10 | –7 | –0.5 | +.01 |
| 27 | +7 | 0 | –0.6 | –.03 |
| 28 | +11 | –5 | –1.1 | –.03 |
| Average | +2.6 | –2.4 | –0.82 | –.012 |

*Hydrochlorothiazide plus Benzazepine*

Hydrochlorothiazide 2.5 mg/kg i.v. first then 3.0 mg/kg/hr infusion started at –20 min. and continued during $C_1$—$C_{12}$. The benzazepine was given orally at 5.0 mg/kg (base) after $C_6$.

| Day | Renal Plasma Flow (RPF) Δ % Change | Glomerular Filtration Rate (GFR) Δ % Change | δ % $Na^+$ Excreted | Filtration Fraction Δ |
|---|---|---|---|---|
| 9 | +28 | +2 | +1.4 | –.07 |
| 14 | +18 | +5 | +0.8 | –.04 |
| 15 | +37 | +11 | +0.1 | –.06 |
| 16 | +23 | +9 | +0.5 | –.05 |
| 29 | +30 | +3 | +0.2 | –.07 |
| Average | +27 | +6 | +0.6 | –.058 |
| t | 5.02** | 2.82* | 5.35** | 4.60** |

t .05 = 2.31
t .01 = 3.36
*Statistically significant at .05 level
**Statistically significant at .01 level

6 - Chloro - 7,8 - dihydroxy - 1 - (4 - hydroxyphenyl) - 2,3,4,5 - tetrahydro - 1H - 3 - benzazepine methyl sulphonic acid salt itself at a single dose demonstrated no diuretic effect in the glucose-infused dog protocol. The combination — hydrochlorothiazide and 6 - chloro - 7,8 - dihydroxy - 1 - (4 - hydroxyphenyl) - 3 - benzazepine — does not demonstrate synergistic activity in the phosphate-mannitol infused dog since this test is not as sensitive as is the glucose dog. Mannitol

itself has some proximal natriuretic effect which tends to screen any synergistic effect of the combination.

However, a combination of triamterene (10 mg. p.o.) and the same benzazepine (2.5 mg. p.o.) in the phosphate mannitol infused dog showed both a decrease in potassium execretion compared with the control phase not present with the individual ingredients as well as a phase IV significant reversal of the decrease in the glomerular filtration rate caused by triamterene.

| | | Phase | | |
|---|---|---|---|---|
| Treatment | Control (I) | II | III | IV |
| *Glomerular Filtration Rate* | | | | |
| Triamterene | 56.86 | –3 | –15 | –15** |
| Benzazepine | 61.91 | 1 | –2 | –2 |
| Combination | 53.78 | –6 | 4* | 3 |
| *Potassium Execretion* | | | | |
| Triamterene | 9.68 | –3.65 | –5.44 | –5.61 |
| Benzazepine | 13.02 | –0.93** | –1.59 | 2.02 |
| Combination | 11.73 | –4.02 | –2.85 | –6.74* |

In the parameters presented, the control figures are actual values. Phase II, III and IV are changes from control. Other parameters in the test, such as renal plasma flow, sodium excretion, and chloride excretion, do not show a synergistic effect over control values.

Among the compositions of this invention which are of particular interest are, as the dopaminergic compound, 6 - chloro - 7,8 - dihydroxy - 1 - (*p* - hydroxyphenyl) - 2,3,4,5 - tetrahydro - 1H - 3 - benzazepine, preferably as its methyl sulfonate salt, combined with, as the diuretic compound, (a) hydrochlorothiazide, (preferably as 25 mg) (b) triamterene (50—100 mg), (c) ticrynafen (250—500 mg) or (d) hydrochlorothiazide (25 mg) plus triamterene (50 mg). In these specific oral compositions the quantity of the dopaminergic compound will in general be selected from 25—100 mg. per dosage unit.

When in sustained release form, the oral dose of benzazepine in the compositions is preferably 100—200 mg. to be given once or twice a day. The diuretic component of these compositions will usually be selected from 25—500 mg per dosage unit.

The following Examples illustrate the preparation and use of the compositions of this invention.

Example 1

6 - Chloro - 7,8 - dihydroxy - 1 - (4 - hydroxyphenyl) - 2,3,4,5 - tetrahydro - 1H - 3 - benzazepine hydrobromide (250 g, 0.646 mole) was dissolved in 3500 ml of methanol with stirring (15 min.). This resultant light orange solution was divided in half and each half added to 2000 ml of 5% sodium bicarbonate solution over 15—20 min. with stirring with the temperature at 25°. The gray slurries were combined, stirred for 30 min., filtered and the filter cake washed with 10 l. of water. The wet gray filter cake which is the free base was slurried in 2 l. of methanol and 50.3 ml (0.775 mole, 20% excess) of methyl sulfonic acid added. The mixture was concentrated to dryness at 75°. The off-white solid was triturated with 400 ml boiling of methanol giving a paste to which 1 l. of ethyl acetate is added with vigorous swirling. The slurry was chilled in the refrigerator, filtered, washed with 300 ml of ethyl acetate then 300 ml of ether. The oven-dried white methyl sulfonate salt weighs 220 g (85%), m.p. 272°.

The salt (100 mg) and 25 mg of hydrochlorothiazide are mixed with 125 mg of lactose and filled into a hard gelatin capsule. Administration to a hypertensive patient is from 3—5 times daily orally.

Example 2

A mixture of 4.84 g of 50% of sodium hydride in mineral oil and 70 ml of dry dimethylsulfoxide was stirred at 65—70° for 80 minutes. After dilution with 70 ml of dry tetrahydrofuran, the mixture was cooled to 0° while a solution of 19.0 g (0.093 mole) of trimethylsulfonium iodide in 100 ml of dimethylsulfoxide was added. A solution of 12.6 g (0.0928 mole) of *m*-anisaldehyde in 40 ml of tetrahydrofuran was quickly added. After stirring for 15 minutes at 0° and 1½ hour at 25°, the mixture was poured into 1½ l. of ice/water slurry and extracted well with water. The combined organic layers were washed with brine, dried and concentrated to give 13 g of crude epoxide. This was mixed with 13.0 g of 2 - (2 - chloro - 3,4 - dimethoxyphenyl)ethylamine and heated at 110° for 4 hours. The product was chromatographed over silica gel with 3% methanol/chloroform. The product containing cuts were worked up to give 1.9 g of N - [2 - (2 - chloro - 3,4 - dimethoxyphenyl)-ethyl] - 2 - hydroxy - 2 - (3 - methoxyphenyl)ethylamine, m.p. 95.5—96.5°.

The 3-methoxy substituted hydroxyphenethylamine intermediate (1.7 g) in 25 ml of 48% hydrogen bromide solution was heated at 135—140° for 3 hours. The solvent was evaporated *in vacuo* and the residue dissolved in methyl alcohol/2-propanol. After charcoal treatment, the solvent was evaporated to leave an amber syrup. This was taken into acetonitrile/2-propanol and a white solid separated by addition of ether. Recrystallization from acetonitrile/ether gave 1.2 g of 6 - chloro - 7,8 - dihydroxy - 1 - (3 - hydroxyphenyl) - 2,3,4,5 - tetrahydro - 1H - 3 - benzazepine hydrobromide, m.p. 195—200°.

The salt (150 mg) is mixed with 25 mg of hydrochlorothiazide and 100 mg of lactose then filled into a hard gelatin capsule for oral administration from 2—4 times daily.

### Example 3

2 - Chloro - 3,4 - dimethoxyphenethylamine (1.0 g) was reacted with 0.70 g of *p*-methoxystyrene oxide to give the hydroxyphenethylamine; m.p. 118.5—121°. This compound (2.16 g) was stirred at room temperature in 15 ml of trifluoroacetic acid with 4 drops of conc. sulfuric acid. Working up gave, after purification, on a silica gel column and using chloroform and 10% methanol/chloroform as eluates, the desired 6 - chloro - 7,8 - dimethoxy - 1 - (4 - methoxyphenyl) - 2,3,4,5 - tetrahydro - 1H - 3 - benzazepine (0.78 g), m.p. 143—145°.

This trimethoxy product (0.87 g, 2.50 mmoles) in 25 ml of dry methylene chloride was cooled in an ice-methanol bath as 12.5 ml (25.0 mmoles) of boron tribromide in methylene chloride were added dropwise. After stirring for 4-hours, the mixture was cooled in an ice bath while methanol was carefully added to give 0.37 g, after crystallization from methanol/ethylacetate, of 6 - chloro - 7,8 - dihydroxy - 1 - (4 - hydroxyphenyl) - 2,3,4,5 - tetrahydro - 1H - 3 - benzazepine hydrobromide, m.p. 215°. Treatment with base and then methyl sulfonic acid gave the methyl sulfonic acid salt, m.p. 272° which is the preferred benzazepine species of the claimed compositions.

A solution of 402 mg (1.0 mmoles) of the methyl sulfonic acid salt in 12 ml of methanol and 4 ml of conc. hydrochloric acid was treated with a solution of 251 mg (1.1 mmole) of 2,3 - dichloro - 5,6 - dicyano - 1,4 - benzoquinone (DDQ) in 2 ml of methanol at room temperature. After stirring overnight, the reaction mixture was treated with aqueous sodium bisulfite until the red color was discharged. The solution was concentrated *in vacuo* to about ½ volume. The tan precipitate was collected by filtration and air-dried to give 430 mg of 6,9 - dichloro - 7,8 - dihydroxy - 1 - (4 - hydroxyphenyl) - 2,3,4,5 - tetrahydro - 1H - 3 - benzazepine hydrochloride; m.p. 220—225° (dec.).

The salt (75 mg) is mixed with cyclopenthiazide (1 mg) and lactose (200 mg), filled into a capsule which is administered orally from 2—3 times daily to induce an antihypertensive effect.

The salt (100 mg) is mixed with 25 mg of hydrochlorothiazide and 125 mg of lactose, filled into a capsule which is administered orally from 3—5 times daily.

### Example 4

Substituting a stoichiometric quantity of 2 - fluoro - 3,4 - dimethoxyphenethylamine in the synthetic procedure of Example 2 gave 6 - fluoro - 7,8 - dimethoxy - 1 - (4 - methoxyphenyl) - 2,3,4,5 - tetrahdyro - 1H - 3 - benzazepine as a yellow oil. Hydrolysis with boron tribromide gave 6 - fluoro - 7,8 - dihydroxy - 1 - (4 - hydroxyphenyl) - 2,3,4,5 - tetrahydro - 1H - 3 - benzazepine hydrobromide, m.p. 277° (dec.). Oxidation with a slight excess of 2,3 - dichloro - 5,6 - dicyano - 1,4 - benzoquinone in methanol and treatment with methanolic hydrochloric acid treatment gives 9 - chloro - 6 - fluoro - 7,8 - dihydroxy - 1 - (4 - hydroxyphenyl) - 2,3,4,5 - tetrahydro - 1H - 3 - benzazepine hydrochloride.

Each benzazepine salt (150 mg) and furosemide (50 mg) with 100 mg of lactose are filled into a capsule which is administered orally to a hypertensive patient.

### Example 5

A mixture of 100 g (0.8055 m) of 2,3-dihydroxytoluene, 325 ml of dimethylsulfate and 460 ml of 10% sodium hydroxide solution was taken to pH of 10 using 40% sodium hydroxide. The mixture was heated at reflux for ½ hour. An additional 50 ml of dimethylsulfate was added with 50 ml of 40% sodium hydroxide. Then the reaction mixture was heated at reflux for 40 minutes. This was repeated; then the mixture was cooled and extracted with ethyl ether. The combined ethereal extract was washed with water and alkali, dried and evaporated to give the desired 2,3-dimethoxytoluene in 98% yield.

This material, 149 g (0.97 m), was added to 557 ml of 1,2-dichloroethane and 150 ml of formaldehyde. The mixture was stirred while hydrogen chloride gas was added for 1½ hours. After a reflux period for 1 hour, the mixture was stirred at room temperature overnight. Hydrogen chloride was

then again bubbled through the mixture for 1 hour. The mixture was heated at reflux for 3 hours. After cooling water was added. The aqueous layer was separated. The organic extracts were combined and washed with brine, dried and evaporated to give 2-methyl-3,4-dimethoxybenzyl chloride, m.p. 67—68.5°.

This compound (80 g, 0.398 m) was taken into 300 ml of dimethylformamide and reacted with 22.43 g (0.457 m) of sodium cyanide at reflux for 1½ hours. Water was added to the mixture which was then extracted with ether. The washed and dried ethereal extracts were evaporated. The remaining oil was taken up in 300 ml of ether. The mixture was washed with 30 ml of conc. hydrochloric acid, shaken well then washed with brine, dried and evaporated. The resulting oil in 200 ml of dimethylformamide with 11 g of sodium cyanide was heated on a steam bath for 1 hour. Worked up as above to give 2-methyl-3,4-dimethoxyphenylacetonitrile, m.p. 60—63°.

A mixture of 42 g (0.219 m) of the phenylacetonitrile 100 ml of tetrahydrofuran was mixed with 400 ml of 1 molar diborane in tetrahydrofuran then heated at reflux for 2½ hours. After cooling the mixture was quenched with 150 ml of methanol. After sitting overnight, the mixture was evaporated to dryness. An excess of 10% hydrochloric acid was added followed by heating on the steambath for 2 hours. After cooling, ether was added. The mixture was stirred for ½ hour and evaporated. The remaining aqueous layer was made basic with 10% sodium hydroxide, extracted with ether which was dried and evaporated to give 2-methyl-3,4-dimethoxyphenethylamine.

The amine (23.3 g, 0.119 m) and methyl *p*-methoxymandelate (23.5 g, 0.119 m) were mixed together and heated on the steam bath overnight. Toluene was added to the oily mixture. The toluene was evaporated with the methanol by-product to give the desired amide in quantitative yield.

This product (41 g, 0.1143 m) was dissolved in 508 ml of toluene and 169.7 ml of sodium aluminum bis(methoxy-ethoxy)-hydride (70% in toluene) was added slowly with stirring under niitrogen. The solution was then heated at 96° for 2 hours. After quenching with water and addition of 10% sodium hydroxide, the toluene layer was separated. The aqueous layer was extracted twice with methylene chloride. The organic extracts were combined and evaporated. The residue was taken up in methylene chloride. The extract was washed with water, dried and evaporated. The residue was recrystallized from ethanol-petroleum ether to give the desired N - [2 - (2 - methyl - 3,4 - dimethoxyphenyl)ethyl] - 2 - hydroxy - 2 - (4 - methoxyphenyl)ethylamine, m.p. 128—129°.

This compound (21.5 g, 0.063 m) was mixed with 161.25 ml of trifluoroacetic acid and 5.16 ml of sulfuric acid. The mixture was stirred at room temperature for 3½ hours. Anhydrous sodium acetate (23.3 g) was then added with cooling. The trifluoroacetic acid was evaporated. Water was added to the residue. The aqueous mixture was made basic with ammonium hydroxide with cooling. The mixture was extracted with ethyl acetate. The combined extract was washed with water, dried, evaporated, ether then added and evaporated to give 6 - methyl - 7,8 - dimethoxy - 1 - (4 - methoxyphenyl) - 2,3,4,5 - tetrahydro - 1H - 3 - benzazepine, m.p. 100—111°.

The trimethoxybenzazepine (1.5 g, 0.0046 m) was dissolved in 15.9 ml of methylene chloride and cooled. A solution of boron tribromide (2M in methylene chloride) was added maintaining the temperature below 15°. The mixture was stirred at room temperature for 3 hours, cooled to —20° and 11.4 ml of methanol added. After returning to room temperature, the slurry was concentrated. The residue was triturated with ethyl acetate, cooled and a solid collected. The product, 6 - methyl - 7,8 - dihydroxy - 1 - (4 - hydroxyphenyl) - 2,3,4,5 - tetrahydro - 1H - 3 - benzazepine hydrobromide, was recrystallized from methanol/ethyl acetate as the hemihydrate; m.p. 319—320° (dec.).

The hydrobromide (100 mg), hydrochlorothiazide (25 mg), spironolactone (25 mg) and lactose (100 mg) are mixed and filled into a capsule.

## Example 6

A mixture of 10 g (0.0255 mole) of 6 - bromo - 7,8 - dimethoxy - 1 - (4 - methoxyphenyl) - 2,3,4,5 - tetrahydro - 1H - 3 - benzazepine prepared by direct bromination using bromine in glacial acetic acid and 42 ml of *n*-butyl lithium was reacted to give the 6-lithium derivative which is reacted with dry ice to give 6 - carboxy - 7,8 - dimethoxy - 1 - (4 - methoxyphenyl) - 2,3,4,5 - tetrahydro - 1H - 3 - benzazepine hydrochloride, m.p. 242—245° (dec.).

The trimethoxy product was treated with boron tribromide in methylene chloride to give the trihydroxy hydrobromide (4.5 g) which contained a small amount of the 6-carbomethoxy ester as by-product. This material (3.5 g) was esterified with 300 ml of methanol-hydrogen chloride by heating at reflux for 64 hours. The product was 6 - carbomethoxy - 7,8 - dihydroxy - 1 - (4 - hydroxyphenyl) - 2,3,4,5 - tetrahydro - 1H - 3 - benzazepine hydrochloride, m.p. 226—229°.

The hydrochloride product (150 mg.) and hydrochlorothiazide (25 mg) are combined with 75 mg of filler and placed in a capsule for oral administration three times daily.

## Example 7

6 - Chloro - 7,8 - dihydroxy - 1 - (4 - hydroxyphenyl) - 2,3,4,5 - tetrahydro - 1H - 3 - benzazepine hydrobromide (1.0 g) was slurried in 200 ml of trifluoroacetic acid then 1.29 ml of acetyl bromide was added. The mixture was heated at reflux for 2 hours then stirred for 2 hours. After evaporation to dryness the residue was taken up in benzene and concentrated to give a solid which was

recrystallized from ethylacetatehexane to give 6 - chloro - 7,8 - diacetoxy - 1 - (4 - acetoxyphenyl) 2,3,4,5 - tetrahydro - 1H - 3 - benzazepine hydrobromide; m.p. 214.5—217°, 0.77 g.

The triester (175 mg) and 25 mg of hydrochlorothiazide and 50 mg of lactose are mixed and filled into a capsule. Other triesters are also so prepared and used.

Example 8

| Ingredients | Amounts, mg: |
|---|---|
| 6-Chloro-7,8-dihydroxy-1-(4-hydroxyphenyl)-2,3,4,5-tetrahydro-1H-3-benzazepine methyl sulfonate | 100 |
| Hydrochlorothiazide | 25 |
| Triamterene | 50 |
| Sucrose | 25 |
| Calcium sulfate, dihydrate | 50 |
| Talc | 5 |
| Stearic acid | 3 |
| Starch | 10 |

The active ingredients sucrose and calcium sulfate are mixed, granulated using hot 10% gelatin solution, meshed and dried. After mixing with the remaining ingredients the mixture is compressed into a scored tablet.

Example 9

FORMULA

| Ingredients: | mg/cap. |
|---|---|
| 6-Chloro-7,8-dihydroxy-1-(4-hydroxyphenyl)-2,3,4,5-tetrahydro-1H-3-benzazepine methyl sulfonate | 131.4* |
| Gelatin | 15.0 |
| Microcrystalline Wax | 5.0 |
| Glyceryl Distearate | 25.0 |
| Non-Pareil Sugar Seeds 25/30 Mesh | 123.6 |
| Hydrochlorothiazide | 25.0 |

*Equivalent to 100 mg free base

Commercial ethanol, methylene chloride and water are used in the coating operation but are removed during processing.

1. Place the non-pareil seeds in a coating pan.
2. Make an alcoholic gelatin solution (e.g., 10% gelatin in alcohol).
3. Spray the alcoholic gelatin solution onto the pellets until evenly wetted.
4. Disperse 10% of the benzazepine salt evenly on the wetted pellets.
5. Roll pellets in the pan until they are dry enough to repeat the application of gelatin solution and drug powder. Continue coating in this manner until all of the drug powder has been applied. Roll the pellets until dry.
6. Make up a solution of microcrystalline wax and glyceryl distearate in methylene chloride.

7. Coat the stock medicated pellets from Step #5 with the appropriate amounts of wax-fat materials (Step #6) to give the desired release rate characteristics. Roll the pellets until dry.

8. Several such groups of wax-fat pellets from Step #7 are blended with stock medicated pellets to yield the sustained release mix.

9. Add meshed hydrochlorothiazide.

10. Fill into opaque gelatin capsule.

Administer such a capsule orally to a hypertensive patient 1—3 times daily.

**Claims for the Contracting States: BE CH DE FR GB IT LI LU NL SE**

1. A synergistically active pharmaceutical composition which comprises a compound of formula (I):—

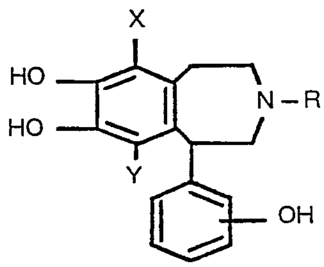

(I)

wherein R is hydrogen or allyl, X is methyl, carbomethoxy or halo, and Y is hydrogen, methyl or halo; its pharmaceutically acceptable acid addition salts or its tri-O-lower alkanoyl esters each lower alkanoyl group having 2—8 carbons: a diuretic compound, and a pharmaceutically acceptable carrier.

2. A composition according to claim 1 wherein Y is hydrogen.

3. A composition according to claim 2 wherein X is chloro.

4. A composition according to any one of claims 1 to 3 wherein R is hydrogen.

5. A composition according to any one of claims 1 to 4 wherein the phenyl hydroxy group is in the para-position.

6. A composition according to claim 1 wherein the compound of the formula (I) is 6 - chloro - 7,8 - dihydroxy - 1 - (*p* - hydroxyphenyl) - 2,3,4,5 - tetrahydro - 1H - 3 - benzazepine as its methyl sulfonic acid salt.

7. A composition according to any one of claims 1 to 6 wherein the quantity of the compound of the formula (I) present is 15—500 mg.

8. A composition according to claim 6 wherein the quantity is 25—100 mg.

9. A composition according to any of claims 1 to 8 wherein the diuretic is a saluretic.

10. A composition according to any of claims 1 to 9 wherein the diuretic is a diluting segment diuretic.

11. A composition according to any of claims 1 to 8 wherein the diuretic is hydrochlorothiazide, triamterene or ticrynafen.

12. A composition according to any of claims 1 to 11 wherein the quantity of diuretic present is 0.5—500 mg.

13. A composition according to claim 12 wherein the quantity is 25—500 mg.

14. A process for the preparation of a pharmaceutical composition according to any of claims 1 to 13 which comprises bringing into association a diuretic and a compound of the formula (I).

15. A composition according to any of claims 1 to 13 for use in the treatment of hypertension.

16. A compound of the formula (I) as defined in claim 1 and a diuretic for use, co-operatively in the treatment of hypertension.

**Claims for the Contracting State: AT**

1. A process for preparing a synergistically active pharmaceutical composition which comprises bringing into association a diuretic compound and a compound of formula (I):—

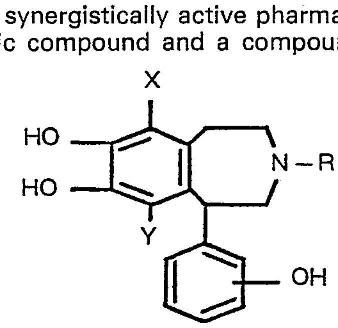

(I)

wherein R is hydrogen or allyl, X is methyl, carbomethoxy or halo, and Y is hydrogen, methyl or halo; its pharmaceutically acceptable acid addition salts or tri-O-lower alkanoyl esters each lower alkanoyl group having 2—8 carbons, and a pharmaceutically acceptable carrier.

2. A process according to claim 1 wherein Y is hydrogen.

3. A process according to claim 2 wherein X is chloro.

4. A process according to any one of claims 1 to 3 wherein R is hydrogen.

5. A process according to any one of claims 1 to 4 wherein the phenyl hydroxy group is in the para-position.

6. A process according to claim 1 wherein the compound of the formula (I) is 6 - chloro - 7,8 - dihydroxy - 1 - (*p* - hydroxyphenyl) - 2,3,4,5 - tetrahydro - 1H - 3 - benzazepine as its methyl sulfonic acid salt.

7. A process according to any one of claims 1 to 6 wherein the quantity of the compound of the formula (I) present is 15—500 mg.

8. A process according to claim 7 wherein the quantity is 25—100 mg.

9. A process according to any of claims 1 to 8 wherein the diuretic is a saluretic.

10. A process according to any of claims 1 to 9 wherein the diuretic is a diluting segment diuretic.

11. A process according to any of claims 1 to 8 wherein the diuretic is hydrochlorothiazide, triamterene or ticrynafen.

12. A process according to any of claims 1 to 11 wherein the quantity of diuretic present is 0.5—500 mg.

13. A process according to claim 12 wherein the quantity is 25—500 mg.

**Revendications pour les Etats contractants: BE CH DE FR GB IT LI LU NL SE**

1. Composition pharmaceutique synergiquement active qui comprend un composé de formule (I):

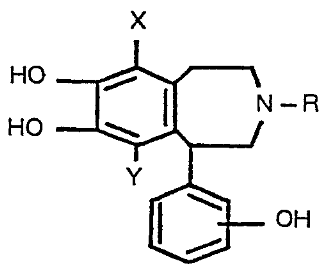

(I)

dans laquelle R représente un atome d'hydrogène ou un groupe allyle X représente un groupe méthyle, carbométhoxy ou halo et Y représente un atome d'hydrogène, un groupe méthyle ou halo; ses sels d'addition avec les acides pharmaceutiquement acceptables ou ses esters tri-O-alcanoyliques inférieurs, chaque groupe alcanoyle inférieur ayant de 2 à 8 atomes de carbone; un composé diurétique et un véhicule pharmaceutiquément acceptable.

2. Composition suivant la revendication 1, dans laquelle Y représente un atome d'hydrogène.

3. Composition suivant la revendication 2, dans laquelle X représente un groupe chloro.

4. Composition suivant l'une quelconque des revendications 1 à 3, dans laquelle R représente un atome d'hydrogène.

5. Composition suivant l'une quelconque des revendicatons 1 à 4, dans laquelle le groupe hydroxy phénolique est en position para.

6. Composition suivant la revendication 1, dans laquelle le composé de la formule (I) est la 6 - chloro - 7,8 - dihydroxy - 1 - (*p* - hydroxyphényl) - 2,3,4,5 - tétrahydro - 1H - 3 - benzazépine sous la forme de son sel d'acide méthylsulfonique.

7. Composition suivant l'une quelconque des revendications 1 à 6, dans laquelle la quantité du composé de la formule (I) en question varie de 15—500 mg.

8. Composition suivant la revendication 6, dans laquelle la quantité varie de 25 à 100 mg.

9. Composition suivant l'une quelconque des revendications 1 à 8, dans laquelle le diurétique est un salidiurétique.

10. Composition suivant l'une quelconque des revendications 1 à 9, dans laquelle le diurétique est un diurétique dit à segment de dilution.

11. Composition suivant l'une quelconque des revendications 1 à 8, dans laquelle la diurétique est l'hydrochlorothiazide, le triamtérène ou le ticrynafen.

12. Composition suivant l'une quelconque des revendications 1 à 11, dans laquelle la quantité de diurétique en question varie de 0,5 à 500 mg.

13. Composition suivant la revendication 12, dans laquelle la quantité varie de 25 à 500 mg.

14. Procédé de préparation d'une composition pharmaceutique suivant l'une quelconque des revendications 1 à 13 qui consiste à associer un diurétique et un composé de formule (I).

15. Composition suivant l'une quelconque des revendications 1 à 13 destinée à servir dans le traitement de l'hypertension.

16. Composé de formule I tel que défini dans la revendication 1 et un diurétique destinés à servir coopérativement dans le traitement de l'hypertension.

**Revendications pour l'Etat contractant: AT**

1. Procédé de préparation d'une composition pharmaceutique synergiquement active qui consiste à associer un composé diurétique et un composé de formule (I):

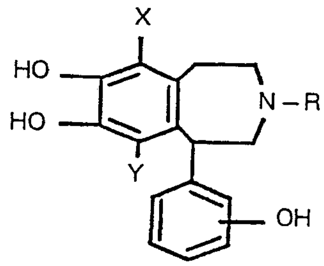

(I)

dans laquelle R représente un atome d'hydrogène ou un groupe allyle, X représente un groupe méthyle, carbométhoxy ou halo et Y représente un atome d'hydrogène, un groupe méthyle ou halo; ses sels d'addition avec les acides pharmaceutiquement acceptables ou ses esters tri-O-alcanoyliques inférieurs, chaque groupe alcanoyle inférieur ayant de 2 à 8 atomes de carbone et un véhicule pharmaceutiquement acceptable.

2. Procédé suivant la revendication 1, dans laquelle Y représente un atome d'hydrogène.

3. Procédé suivant la revendication 2, dans laquelle X représente un groupe chloro.

4. Procédé suivant l'une quelconque des revendications 1 à 3, dans laquelle R représente un atome d'hydrogène.

5. Procédé suivant l'une quelconque des revendications 1 à 4, dans laquelle le groupe hydroxy phénolique est en position para.

6. Procédé suivant la revendication 1, dans laquelle le composé de la formule (I) est la 6 - chloro - 7,8 - dihydroxy - 1 - (*p* - hydroxyphényl) - 2,3,4,5 - tétrahydro - 1H - 3 - benzazépine sous la forme de son sel d'acide méthylsulfonique.

7. Procédé suivant l'une quelconque des revendications 1 à 6, dans laquelle la quantité du composé de la formule (I) en question varie de 15—500 mg.

8. Procédé suivant la revendication 6, dans laquelle la quantité varie de 25 à 100 mg.

9. Procédé suivant l'une quelconque des revendications 1 à 8, dans laquelle le diurétique est un salidiurétique.

10. Procédé suivant l'une quelconque des revendications 1 à 9, dans laquelle le diurétique est un diurétique dit à segment de dilution.

11. Procédé suivant l'une quelconque des revendications 1 à 8, dans laquelle le diurétique est l'hydrochlorothiazide, le triamtérène ou le ticrynafen.

12. Procédé suivant l'une quelconque des revendications 1 à 11, dans laquelle la quantité de diurétique en question varie de 0,5 à 500 mg.

13. Procédé suivant la revendication 12, dans laquelle la quantité varie de 25 à 500 mg.

**Patentansprüche für die Vertragsstaaten: BE CH DE FR GB IT LI LU NL SE**

1. Ein synergistisch wirkendes Arzneimittel, das eine Verbindung der Formel I

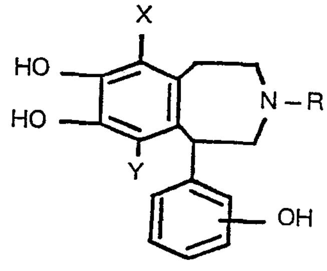

(I)

in der R Wasserstoff oder Allyl, X Methyl, Carbomethoxy oder Halogen und Y Wasserstoff, Methyl oder Halogen ist, pharmazeutisch verträgliche Säureadditionssalze oder Tri-O-niederalkanoylester mit 2 bis 8 Kohlenstoffatomen in jedem Niederalkanoylrest, und ein Diuretikum sowie einen pharmazeutisch verträglichen Träger umfaßt.

2. Ein Mittel nach Anspruch 1, wobei Y Wasserstoff ist.
3. Ein Mittel nach Anspruch 2, wobei X Chlor ist.
4. Ein Mittel nach einem der Ansprüche 1 bis 3, wobei R Wasserstoff ist.
5. Ein Mittel nach einem der Ansprüche 1 bis 4, wobei die Phenyl-Hydroxylgruppe in p-Stellung ist.
6. Ein Mittel nach Anspruch 1, wobei die Verbindung der Formel I 6 - Chlor - 7,8 - dihydroxy - 1 - (p - hydroxyphenyl) - 2,3,4,5 - tetrahydro - 1H - 3 - benzazepin in Form seines Methylsulfonsäuresalzes ist.
7. Ein Mittel nach einem der Ansprüche 1 bis 6, wobei die Menge der anwesenden Verbindung der Formel I 15 bis 500 mg beträgt.
8. Ein Mittel nach Anspruch 6, wobei die Menge 25 bis 100 mg beträgt.
9. Ein Mittel nach einem der Ansprüche 1 bis 8, wobei das Diuretikum ein Saluretikum ist.
10. Ein Mittel nach einem der Ansprüche 1 bis 9, wobei das Diuretikum ein verdünnendes Segment-Diuretikum ist.
11. Ein Mittel nach einem der Ansprüche 1 bis 8, wobei das Diuretikum Hydrochlorthiazid, Triamteren oder Ticrynafen ist.
12. Ein Mittel nach einem der Ansprüche 1 bis 11, wobei die Menge an anwesendem Diuretikum 0,5 bis 500 mg beträgt.
13. Ein Mittel nach Anspruch 12, wobei die Menge 25 bis 500 mg beträgt.
14. Ein Verfahren zur Herstellung eines Arzneimittels nach einem der Ansprüche 1 bis 13, das Zusammenbringen eines Diuretikums und einer Verbindung der Formel I umfaßt.
15. Ein Mittel nach einem der Ansprüche 1 bis 13 zur Verwendung bei der Behandlung von Bluthochdruck.
16. Eine Verbindung der Formel I gemäß Anspruch 1 und ein Diuretikum zur Verwendung in Zusammenwirkung bei der Behandlung von Bluthochdruck.

**Patentansprüche für den Vertragsstaat: AT**

1. Ein Verfahren zur Herstellung eines synergistisch wirkenden Arzneimittels, das Zusammenbringen eines Diuretikums mit einer Verbindung der Formel I

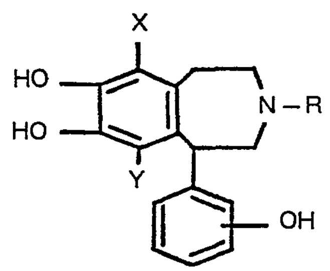

(I)

in der R Wasserstoff oder Allyl, X Methyl, Carbomethoxy oder Halogen und Y Wasserstoff, Methyl oder Halogen ist, pharmazeutisch verträglichen Säureadditionssalzen oder Tri-O-niederalkanoylestern davon mit 2 bis 8 Kohlenstoffatomen in jedem Niederalkanoylrest, sowie einem pharmazeutisch verträglichen Träger umfaßt.
2. Ein Verfahren nach Anspruch 1, wobei Y Wasserstoff ist.
3. Ein Verfahren nach Anspruch 2, wobei X Chlor ist.
4. Ein Verfahren nach einem der Ansprüche 1 bis 3, wobei R Wasserstoff ist.
5. Ein Verfahren nach einem der Ansprüche 1 bis 4, wobei die Phenyl-Hydroxylgruppe in p-Stellung ist.
6. Ein Verfahren nach Anspruch 1, wobei die Verbindung der Formel I 6 - Chlor - 7,8 - dihydroxy - 1 - (p - hydroxyphenyl) - 2,3,4,5 - tetrahydro - 1H - 3 - benzazepin in Form seines Methylsulfonsäuresalzes ist.
7. Ein Verfahren nach einem der Ansprüche 1 bis 6, wobei die Menge der anwesenden Verbindung der Formel I 15 bis 500 mg beträgt.
8. Ein Verfahren nach Anspruch 7, wobei die Menge 25 bis 100 mg beträgt.
9. Ein Verfahren nach einem der Ansprüche 1 bis 8, wobei das Diuretikum ein Saluretikum ist.
10. Ein Verfahren nach einem der Ansprüche 1 bis 9, wobei das Diuretikum ein verdünnendes Segment-Diuretikum ist.
11. Ein Verfahren nach einem der Ansprüche 1 bis 8, wobei das Diuretikum Hydrochlorthiazid, Triamteren oder Ticrynafen ist.
12. Ein Verfahren nach einem der Ansprüche 1 bis 11, wobei die Menge an anwesendem Diuretikum 0,5 bis 500 mg beträgt.
13. Ein Verfahren nach Anspruch 12, wobei die Menge 25 bis 500 mg beträgt.